# EUROPEAN PATENT SPECIFICATION

(11) **EP 1 035 827 B1**
(45) Date of publication and mention of the grant of the patent: **03.05.2006**
(21) Application number: 98965692.1
(22) Date of filing: 16.11.1998
(51) Int. Cl.: A61Q 5/00

(54) **HAIR TREATMENT COMPOSITION**
HAARPFLEGEZUSAMMENSETZUNG
COMPOSITION DE TRAITEMENT POUR LES CHEVEUX

(30) Priority: 04.12.1997 AR 1005697; 05.03.1998 GB 9804717
(43) Date of publication of application: 20.09.2000
(73) Proprietor: UNILEVER PLC, London EC4P 4BQ (GB); UNILEVER N.V., 3013 AL Rotterdam (NL)
(72) Inventor: BERTOLOSSO, Gerardo, 1427-Buenos Aires (AR); MAVROPOULOU, Maria, 1427-Buenos Aires (AR); MURRAY, Andrew, Malcolm, Wirral Merseyside L63 3JW (GB)
(74) Representative: Elliott, Peter William
(86) International application number: PCT/EP1998/007533
(87) International publication number: WO 1999/029286

(56) References cited:
- WO-A-97/32917
- GB-A- 2 173 515
- GB-A- 2 307 407
- GB-A- 2 316 615

## Description

### FIELD OF THE INVENTION

This invention relates to hair treatment compositions, and more particularly to hair treatment compositions containing emulsified particles of silicone, which compositions condition the hair leaving it softer and more manageable.

### BACKGROUND AND PRIOR ART

The use of silicones as conditioning agents in cosmetic formulations is well known and widely documented in the patent literature. Generally, dispersed droplets of the silicone oil are suspended in the composition, which is then applied to the hair to deposit the silicone material on the hair shaft.

US 5,198,209 (Amway Corp) exemplifies conditioning shampoos with cleansing surfactant and a combination of dimethicone and trimethylsilylamodimethicone, and mentions that superior conditioning benefits are obtainable when an amino functionalised silicone is used with the polydimethylsiloxane.

L'Oreal EP 0 811 371 describes cleansing compositions for hair comprising surfactant and a conditioning system comprising (i) cationic polymer (ii) amino functionalised silicone (iii) insoluble non-amino functionalised silicone of viscosity less than or equal to 100,000 cst. The composition is said to improve wet and dry combing, manageability, softness and smoothness. Preferred is a mixture of DC939 amodimethicone emulsion and DC200 (60,000 cst) dimethicone fluid.

A problem encountered with silicone shampoo formulations is that the conditioning performance may be insufficient for many people, particularly in regions such as Japan and South East Asia where consumers desire a high level of conditioning and a "weighty" feel to their hair.

We have now found that superior conditioning performance over dimethicone-based systems such as described in the prior art can be obtained by utilising emulsified hydroxyl functionalised silicone in combination with amino functionalised silicone. This is surprising, given that articles such as "Organofunctional Silicones for Personal Care Applications", (Wendel, Samuel R and DiSapio, Alfred *J. Cosmetics & Toiletries* vol 98 May 1983, pp 103-106) have generally taught away from the use of hydroxyl functionalised silicones, such as dimethiconol, in hair treatment compositions.

Furthermore, a frequently encountered problem with consumers is "combination" hair, i.e. a combination of oily hair roots (the first 5 to 10 cm of the hair fibre) and dry hair ends. Often this is exacerbated by over washing and repeated harsh mechanical and/or chemical treatments such as heat-styling, bleaching, perming and colouring. Typically, hair treatment formulations incorporating conditioning agents such as silicones are indiscriminate in their conditioning action. This means that whereas such formulations may be effective in softening and detangling dry brittle ends, they will at the same time tend to over-condition the oily roots where less conditioning is required, leading to dullness and greasy feel in this area.

We have found that compositions of the invention also show superior selectivity in conditioning performance. The combination of emulsified hydroxyl functionalised silicone and amino functionalised silicone gives demonstrably improved targeting towards those areas of the hair, such as the ends, where conditioning is needed most.

### SUMMARY OF THE INVENTION

The invention provides aqueous hair treatment compositions as disclosed in claims 1 or 2.

In a further aspect, the invention provides the use of a composition as defined above for the selective conditioning of combination hair.

### DETAILED DESCRIPTION OF THE INVENTION

### (i) Amino functionalised silicone

By "amino functionalised silicone" is meant a silicone containing at least one primary, secondary or tertiary amine group, or a quaternary ammonium group.

Examples include:
(i) polysiloxanes having the CTFA designation "amodimethicone", and the general formula:

   HO-[Si(CH₃)₂-O-]ₓ-[Si(OH) (CH₂CH₂CH₂-NH-CH₂CH₂NH₂)-O-]_{y}-H

   in which x and y are numbers depending on the molecular weight of the polymer, generally such that the molecular weight is between about 5,000 and 500,000.
(ii) polysiloxanes having the general formula:

   R'ₐG₃₋ₐ-Si(OSiG₂)ₙ-(OSiG_{b}R'_{2-b})ₘ-O-SiG₃₋ₐ-R'ₐ

   in which:
   G is selected from H, phenyl, OH or C₁₋₈ alkyl, e.g. methyl;
   a is 0 or an integer from 1 to 3, preferably 0;
   b is 0 or 1, preferably 1;
   m and n are numbers such that (m + n) can range from 1 to 2000, preferably from 50 to 150;
   m is a number from 1 to 2000, preferably from 1 to 10;
   n is a number from 0 to 1999, preferably from 49 to 149, and
   R' is a monovalent radical of formula -C_{q}H_{2q}L in which q is a number from 2 to 8 and L is an amino functional group selected from the following:

      -NR"-CH₂-CH₂-N(R")₂

      -N(R")₂

      -N⁺(R")₃A⁻

      -N⁺H(R")₂ A⁻

      -N⁺H₂(R") A⁻

      -N(R")-CH₂-CH₂-N⁺H₂(R") A⁻

      in which R" is selected from H, phenyl, benzyl, or a saturated monovalent hydrocarbon radical, e.g. C₁₋₂₀ alkyl, and
   A is a halide ion, e.g. chloride or bromide.
      Suitable amino functionalised silicones corresponding to the above formula include those polysiloxanes termed "trimethylsilylamodimethicone" as depicted below, and which are sufficiently water insoluble so as to be useful in compositions of the invention:

      Si(CH₃)₃-O-[Si(CH₃)₂-O-]ₓ-[Si(CH₃) (R-NH-CH₂CH₂NH₂)-O-]_{y}-Si(CH₃)₃

      wherein x + y is a number from about 50 to about 500, and wherein R is an alkylene group having from 2 to 5 carbon atoms. Preferably, the number x + y is in the range of from about 100 to about 300.
(iii) quaternary silicone polymers having the general formula:

   {(R¹) (R²) (R³) N⁺ CH₂CH(OH)CH₂O(CH₂)₃[Si(R⁴) (R⁵)-O-]ₙ-Si(R⁶) (R⁷)-(CH₂)₃-O-CH₂CH (OH)CH₂N⁺(R⁸) (R⁹) (R¹⁰)} (X⁻) ₂

   wherein R¹ and R¹⁰ may be the same or different and may be independently selected from H, saturated or unsaturated long or short chain alk(en)yl, branched chain alk(en)yl and C₅-C₈ cyclic ring systems;
   R² thru' R⁹ may be the same or different and may be independently selected from H, straight or branched chain lower alk(en)yl, and C₅-C₈ cyclic ring systems;
   n is a number within the range of about 60 to about 120, preferably about 80, and
   X⁻ is preferably acetate, but may instead be for example halide, organic carboxylate, organic sulphonate or the like.

Suitable quaternary silicone polymers of this class are described in EP-A-0 530 974.

Amino functionalised silicones suitable for use in the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole % since we have found that too high an amine concentration can be detrimental to total silicone deposition and therefore conditioning performance.

The viscosity of the amino functionalised silicone is not particularly critical and can suitably range from about 100 to about 500,000 cst.

Specific examples of amino functionalised silicones suitable for use in the invention are the aminosilicone oils DC2-8220, DC2-8166, DC2-8466, and DC2-8950-114 (all ex Dow Corning), and GE 1149-75, (ex General Electric Silicones).

Also suitable are emulsions of amino functionalised silicone oils with non ionic and/or cationic surfactant.

Suitably such pre-formed emulsions will have an average amino functionalised silicone particle size in the hair treatment composition of less than 30, preferably less than 20, more preferably less than 10 microns. We have found that reducing the particle size generally improves conditioning performance. Most preferably the average amino functionalised silicone particle size in the hair treatment composition is less than 2 microns, ideally it ranges from 0.01 to 1 micron. Silicone emulsions having an average silicone particle size of ≤ 0.15 microns are generally termed microemulsions.

Particle size may be measured by means of a laser light scattering technique, using a 2600D Particle Sizer from Malvern Instruments.

Pre-formed emulsions of amino functionalised silicone are available from suppliers of silicone oils such as Dow Corning and General Electric. Specific examples include DC929 Cationic Emulsion, DC939 Cationic Emulsion, DC949 Cationic Emulsion, and the non-ionic emulsions DC2-7224, DC2-8467 and DC2-8154 (all ex Dow Corning).

An example of a quaternary silicone polymer useful in the present invention is the material K3474, ex Goldschmidt.

### (ii) Emulsified, hydroxyl functionalised silicone

The hair treatment composition of the invention comprises a hydroxyl functionalised silicone. The silicone is insoluble in the aqueous matrix of the hair treatment composition and so is present in an emulsified form, with the silicone present as dispersed particles.

Suitable silicones include polydimethyl siloxanes having hydroxyl end groups, which have the CTFA designation dimethiconol. Also suitable for use in compositions of the invention are hydroxyl functionalised silicone gums having a slight degree of cross-linking, as are described for example in WO 96/31188. These materials can impart body, volume and stylability to hair, as well as good wet and dry conditioning.

Various methods of making emulsions of particles of silicones for use in the invention are available and are well known and documented in the art. For example, emulsions may be prepared by high shear mechanical mixing of the silicone and water, or by emulsifying the silicone with water and an emulsifier (mixing the silicone into a heated solution of the emulsifier for instance), or by a combination of mechanical and chemical emulsification. A further suitable technique for preparation of emulsions of particles of silicones is emulsion polymerisation. Emulsion polymerised silicones as such are described in US 2 891 820 (Hyde), US 3 294 725 (Findlay) and US 3 360 491 (Axon).

Suitable silicone emulsions for use in the invention are commercially available in a pre-emulsified form. This is particularly preferred since the pre-formed emulsion can be incorporated into the hair treatment composition by simple mixing. Pre-formed emulsions are available from suppliers of silicone oils such as Dow Corning, General Electric, Union Carbide, Wacker Chemie, Shin Etsu, Toshiba, Toyo Beauty Co, and Toray Silicone Co.

The viscosity of the silicone itself (not the emulsion or the final hair treatment composition) is typically at least 10,000 cst. In general we have found that conditioning performance increases with increased viscosity. Accordingly, the viscosity of the silicone itself is preferably at least 60,000 cst, most preferably at least 500,000 cst, ideally at least 1,000,000 cst. Preferably the viscosity does not exceed 10⁹ cst for ease of formulation. Viscosity can be measured by means of a glass capillary viscometer as set out further in Dow Corning Corporate Test Method CTM004 July 20 1970.

Emulsified hydroxyl functionalised silicones for use in hair treatment compositions of the invention will also typically have an average silicone particle size in the hair treatment composition of less than 30, preferably less than 20, more preferably less than 10 microns. Again, we have found that reducing the particle size generally improves conditioning performance. Most preferably the average silicone particle size of the emulsified hydroxyl functionalised silicone in the hair treatment composition is less than 2 microns, ideally it ranges from 0.01 to 1 micron.

Particle size may be measured by means of a laser light scattering technique, using a 2600D Particle Sizer from Malvern Instruments.

Examples of suitable pre-formed emulsions include emulsions DC2-1766, DC2-1784, and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

### Silicone Ratios

We have found that superior conditioning and improved selectivity of conditioning performance can be achieved by combining emulsified hydroxyl functionalised silicone with amino functionalised silicone in a hair treatment composition.

The weight ratio of amino functionalised silicone to hydroxyl functionalised silicone should generally be 1:2 or less. Suitably, the ratio of amino functionalised silicone to hydroxyl functionalised silicone ranges from 1:2 to 1:20, preferably 1:3 to 1:20, more preferably 1:3 to 1:8, optimally around 1:4.

We have found that if too much amino functionalised silicone is included in the composition relative to emulsified hydroxyl functionalised silicone, then this may in some circumstances have a detrimental effect on conditioning performance. This has been found to be the case, for example, where rinse-off cleansing shampoo-type compositions are concerned.

### Silicone Levels

The total amount of silicone (amino functionalised and hydroxyl functionalised) incorporated into the hair treatment compositions of the invention depends on the level of conditioning desired and the material used. A preferred amount is from 0.01 to about 10% by weight of the total composition although these limits are not absolute. The lower limit is determined by the minimum level to achieve conditioning and the upper limit by the maximum level to avoid making the hair and/or skin unacceptably greasy.

We have found that a total amount of silicone of from 0.3 to 5%, preferably 0.5 to 3%, by weight of the total composition is a suitable level.

### (iii) Product Form

Hair treatment compositions according to the invention may suitably take the form of shampoos, conditioners, sprays, mousses or lotions. Preferred hair treatment composition forms are shampoos and conditioners.

### Shampoo Compositions

A particularly preferred hair treatment composition in accordance with the invention is a shampoo composition.

### - Cleansing Surfactant

Such a shampoo composition will comprise one or more cleansing surfactants which are cosmetically acceptable and suitable for topical application to the hair. Further surfactants may be present as an additional ingredient if sufficient for cleansing purposes is not provided as emulsifying agent for oily or hydrophobic components (such as silicones) which may typically be present in the shampoo.

It is preferred that shampoo compositions of the invention comprise at least one further surfactant (in addition to that used as emulsifying agent) to provide a cleansing benefit.

Suitable cleansing surfactants, which may be used singularly or in combination, are selected from anionic, amphoteric and zwitterionic surfactants, and mixtures thereof. The cleansing surfactant may be the same surfactant as the emulsifier, or may be different.

Examples of anionic surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic surfactants for use in shampoos of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, triethanolamine monolauryl phosphate, sodium lauryl ether sulphate 1 EO, 2EO and 3EO, ammonium lauryl sulphate and ammonium lauryl ether sulphate 1EO, 2EO and 3EO.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

The shampoo composition can also include co-surfactants, to help impart aesthetic, physical or cleansing properties to the composition. A preferred example is a nonionic surfactant, which can be included in an amount ranging from 0% to about 5% by weight based on total weight.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionics include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

The total amount of surfactant (including any co-surfactant, and/or any emulsifying agent) in shampoo compositions of the invention is generally from 0.1 to 50% by weight, preferably from 5 to 30%, more preferably from 10% to 25% by weight of the total shampoo composition.

### - Cationic Polymer

A cationic polymer as disclosed in claims 1 or 2 is an essential ingredient in shampoo compositions of the invention, for enhancing conditioning performance of the shampoo. Typically such a polymer enhances deposition of conditioning components such as silicone from the shampoo composition onto the intended site during use, i.e. the hair and/or the scalp.

The cationic polymer may be a homopolymer or be formed from two or more types of monomers. The molecular weight of the polymer will generally be between 5 000 and 10 000 000, typically at least 10 000 and preferably in the range 100 000 to about 2 000 000. The polymers will have cationic nitrogen containing groups such as quaternary ammonium or protonated amino groups, or a mixture thereof.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose (e.g. as described in U.S. Patent 3,958,581).

Particularly suitable is a cationic guar gum derivative, such as guar hydroxypropyltrimonium chloride (Commercially available from Rhone-Poulenc in their JAGUAR trademark series).

Examples are JAGUAR C13S, and JAGUAR CB289, which have a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

The cationic polymer is selected from cationic cellulose and cationic guar derivatives. Particularly preferred cationic polymers are JAGUAR C13S, JAGUAR CB289, JAGUAR C15, JAGUAR C17 and JAGUAR C16 and JAGUAR C162.

### Conditioners

Compositions in accordance with the invention may also be formulated as conditioners for the treatment of hair (typically after shampooing) and subsequent rinsing.

### - Conditioning Surfactant

Such a conditioner will comprise one or more conditioning surfactants which are cosmetically acceptable and suitable for topical application to the hair.

Suitable conditioning surfactants are selected from cationic surfactants, used singly or in admixture. Examples include quaternary ammonium hydroxides or salts thereof, e.g. chlorides.

Suitable cationic surfactants for use in hair conditioners of the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as DEHYQUART, ex Henkel.

In conditioners of the invention, the level of cationic surfactant is preferably from 0.01 to 10%, more preferably 0.05 to 5%, most preferably 0.1 to 2% by weight of the composition.

### - Fatty Alcohol

Conditioners of the invention advantageously incorporate a fatty alcohol material. The combined use of fatty alcohol materials and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 20. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol material in conditioners of the invention is conveniently from 0.01 to 10%, preferably from 0.1 to 5% by weight of the composition. The weight ratio of cationic surfactant to fatty alcohol is suitably from 10:1 to 1:10, preferably from 4:1 to 1:8, optimally from 1:1 to 1:4.

### (iv) Optional Ingredients

Compositions of this invention may contain any other ingredient normally used in hair treatment formulations. These other ingredients may include viscosity modifiers, preservatives, colouring agents, polyols such as glycerine and polypropylene glycol, chelating agents such as EDTA, antioxidants such as vitamin E acetate, fragrances, antimicrobials and sunscreens. Each of these ingredients will be present in an amount effective to accomplish its purpose. Generally these optional ingredients are included individually at a level of up to about 5% by weight of the total composition.

Preferably, compositions of this invention also contain adjuvants suitable for hair care. Generally such ingredients are included individually at a level of up to 2%, preferably up to 1%, by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine.
(ii) hair fibre benefit agents. Examples are:
   ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.
   fatty acids, for cuticle repair and damage prevention. Examples are branched chain fatty acids such as 18-methyleicosanoic acid and other homologues of this series, straight chain fatty acids such as stearic, myristic and palmitic acids, and unsaturated fatty acids such as oleic acid, linoleic acid, linolenic acid and arachidonic acid. A preferred fatty acid is oleic acid. The fatty acids may be added singly, as mixtures, or in the form of blends derived from extracts of, e.g. lanolin.

Mixtures of any of the above active ingredients may also be used.

The invention is further illustrated by way of the following examples, in which all percentages quoted are by weight based on total weight unless otherwise stated:

### EXAMPLES

### Example 1

A shampoo composition was prepared by mixing the following components in the amounts stated:

### Ingredient

| | % wt |
|---|---|
| Sodium lauryl ether sulphate (2EO) | 14.0 |
| Cocamidopropyl betaine | 2.0 |
| Hydroxyl functionalised silicone⁽¹⁾ | 1.5 |
| Sodium chloride | 1.5 |
| Amino-functionalised silicone⁽²⁾ | 0.5 |
| Carbopol 980 ⁽³⁾ | 0.4 |
| Jaguar C13S⁽⁴⁾ | 0.1 |
| Preservative, perfume, colour | q.s. |
| Water to | 100.0 |

| | |
|---|---|
| ⁽¹⁾ Hydroxyl functionalised silicone was included as DC2-1784 from Dow Corning Ltd., an emulsion (50% a.i.) of dimethiconol (1 million cst, 0.5 micron particle size) in anionic surfactant (TEA-dodecylbenzenesulfonate). | |
| ⁽²⁾ Amino functionalised silicone was included as DC929 from Dow Corning Ltd., an emulsion (35% a.i.) of amodimethicone in cationic surfactant(tallowtrimonium chloride) and nonionic surfactant (nonoxynol-10). | |
| ⁽³⁾ Carbopol 980 is a cross-linked polyacrylate available from B F Goodrich. | |
| ⁽⁴⁾ Jaguar C13S is guar hydroxypropyltrimonium chloride available from Rhodia (formerly Rhone-Poulenc) | |

The shampoo was prepared by a simple cold mixing process at a temperature not exceeding 40°C. Jaguar C13S and the minor ingredients were added to sodium lauryl ether sulphate (2EO) solution, and mixed until homogenised. Cocamidopropyl betaine solution was then added along with the silicone combination. Finally sodium chloride was added and the final composition mixed until homogenised.

### Example 2 and Comparative Example A

Two shampoo compositions were made up having ingredients as shown in the following Table:

| **INGREDIENT** | **% weight** | |
|---|---|---|
| | **Comparative Example A** | **Example 2** |
| Sodium lauryl ether sulphate (2EO) | 14.0 | 14.0 |
| Cocamidopropyl betaine | 2.0 | 2.0 |
| Jaguar C13S | 0.1 | 0.1 |
| Pearliser⁽⁵⁾ | 6.0 | 6.0 |
| Formalin | 0.1 | 0.1 |
| DC 949⁽⁶⁾ | 1.0 | 1.0 |
| DC 200 (60,000cst) ⁽⁷⁾ | 2.0 | --- |
| X2-1766⁽⁸⁾ | --- | 2.0 |
| NaCl | 0.9 | 0.9 |
| H₂O | to 100 | to 100 |

| | | |
|---|---|---|
| ⁽⁵⁾ EUPERLAN PK3000, ex Henkel | | |
| ⁽⁶⁾ An emulsion (35% a.i.) of aminoethylaminopropyl dimethylsiloxane emulsified with alkyltrimethylammonium chloride and polyethoxylated tridecylalcohol, ex Dow Corning | | |
| ⁽⁷⁾ Dimethicone fluid, viscosity 60,000 cst, ex Dow Corning | | |
| ⁽⁸⁾ An emulsion (60% a.i.) of dimethiconol (1 million cst, 0.5 micron particle size) in anionic surfactant (sodium lauryl sulphate), ex Dow Corning | | |

| The shampoos of Example 2 and Comparative Example A were subjected to a panellist evaluation for various wet and dry conditioning attributes. The panellist preferences are shown in the following Table:**Attribute** | **Example 2** | **Comparative Example A** |
|---|---|---|
| *WET FEEL* | | |
| smoothness | 79 | 21 |
| ease of comb | 83 | 17 |

| *DRY FEEL* | | |
|---|---|---|
| smoothness | 75 | 25 |
| ease of comb | 83 | 17 |
| lack of fly-away | 85 | 15 |

Clearly the composition of the invention (with hydroxyl functionalised silicone and amino functionalised silicone) outperformed the composition of the Comparative Example over all attributes tested.

### Example 3

The composition of Example 1 was subjected to the following *in vitro* test to establish selective conditioning ability:

An 0.25g hair switch was washed with a 10% solution in water (by weight based on total weight) of the composition of Example 1. The hair switch was then rinsed with water. After treatment, the root and end regions of the hair switch were analysed separately for silicone deposition by X-ray fluorescence.

The results showed a greater concentration of silicones at the hair ends, i.e. the region which is drier and more damaged *in vivo.*

## Claims

1. An aqueous hair treatment composition which is a shampoo composition comprising, in addition to water:
i) an amino functionalised silicone; and
ii) emulsified particles of an insoluble, hydroxyl functionalised silicone, and from 0.001 to 5% by weight of the total shampoo composition of a cationic deposition polymer selected from cationic cellulose and cationic guar derivatives.

2. An aqueous shampoo composition comprising, in addition to water:
i) at least one surfactant chosen from anionic, nonionic, zwitterionic or amphoteric surfactants or mixtures thereof;
ii) an amino functionalised silicone; and
iii) emulsified particles of an insoluble, hydroxyl functionalised silicone, and from 0.001 to 5% by weight of the total shampoo composition of a cationic deposition polymer selected from cationic cellulose and cationic guar derivatives, in which the weight ratio of amino functionalised silicone
(ii) to hydroxyl functionalised silicone (iii) is 1:2 or less.

3. A shampoo composition according to claim 1, in which the weight ratio of amino functionalised silicone to hydroxyl functionalised silicone ranges from 1:3 to 1:8, preferably around 1:4.

4. A shampoo composition according to any preceding claim, in which the amino functionalised silicone has a mole % amine functionality in the range of from about 0.1 to about 8.0 mole %, preferably from about 0.1 to about 5.0 mole %, most preferably from about 0.1 to about 2.0 mole %.

5. A shampoo composition according to any preceding claim, in which the amino functionalized silicone is in the form of an emulsion of amino functionalised silicone oil with non ionic and/or cationic surfactant, and in which the average particle size of the amino functionalised silicone in the shampoo composition is less than 2 microns.

6. A shampoo composition according to any preceding claim in which the emulsified hydroxyl functionalised silicone has an average silicone particle size in the shampoo composition of less than 2 microns.

7. A shampoo composition according to any preceding claim, in which the emulsified hydroxyl functionalised silicone has a viscosity (of the silicone itself) of at least 500,000 cst.

8. A shampoo composition according to any preceding claim, in which the total amount of silicone is from 0.3 to 5%, preferably 0.5 to 3%, by weight of the total shampoo composition.

9. The use of a composition as defined in any one of claims 1 to 8 for the selective conditioning of combination hair.

## Patentansprüche

1. Wässrige Haarbehandlungszusammensetzung, die eine Shampoozusammensetzung darstellt, umfassend, zusätzlich zu Wasser:
i) ein Amino-funktionalisiertes Silikon; und
ii) emulgierte Teilchen eines unlöslichen, Hydroxyl-funktionalisierten Silikons und 0,001 bis 5 Gewichtsprozent der gesamten Shampoozusammensetzung eines kationischen Abscheidungspolymers, ausgewählt aus kationischer Cellulose und kationischen Guarderivaten.

2. Wässrige Shampoozusammensetzung, umfassend, zusätzlich zu Wasser:
i) mindestens ein Tensid, ausgewählt aus anionischen, nichtionischen, zwitterionischen oder amphoteren Tensiden oder Gemischen davon;
ii) ein Amino-funktionalisiertes Silikon; und
iii) emulgierte Teilchen eines unlöslichen, Hydroxyl-funktionalisierten Silikons, und 0,001 bis 5 Gewichtsprozent der Gesamtshampoozusammensetzung eines kationischen Abscheidungspolymers, ausgewählt aus kationischer Cellulose und kationischen Guarderivaten, worin das Gewichtsverhältnis von Amino-funktionalisiertem Silikon (ii) zu Hydroxyl-funktionalisiertem Silikon (iii) 1:2 oder weniger ist.

3. Shampoozusammensetzung nach Anspruch 1, worin das Gewichtsverhältnis von Amino-funktionalisiertem Silikon zu Hydroxyl-funktionalisiertem Silikon im Bereich von 1:3 bis 1:8, vorzugsweise etwa 1:4, ist.

4. Shampoozusammensetzung nach einem vorangehenden Anspruch, worin das Amino-funktionalisierte Silikon eine Aminfunktionalität in Molprozent im Bereich von etwa 0,1 bis etwa 8,0 Molprozent, vorzugsweise etwa 0,1 bis etwa 5,0 Molprozent, besonders bevorzugt etwa 0,1 bis etwa 2,0 Molprozent, aufweist.

5. Shampoozusammensetzung nach einem vorangehenden Anspruch, worin das Amino-funktionalisierte Silikon in Form einer Emulsion eines Amino-funktionalisierten Silikonöls mit nichtionischem und/oder kationischem Tensid vorliegt, und worin die mittlere Teilchengräße des Amino-funktionalisierten Silikons in der Shampoozusammensetzung weniger als 2 µm ist.

6. Shampoozusammensetzung nach einem vorangehenden Anspruch, worin das emulgierte Hydroxyl-funktionalisierte Silikon eine mittlere Silikonteilchengröße in der Shampoozusammensetzung von weniger als 2 µm aufweist.

7. Shampoozusammensetzung nach einem vorangehenden Anspruch, worin das emulgierte Hydroxyl-funktionalisierte Silikon eine Viskosität (von dem Silikon selbst) von mindestens 500 000 cSt aufweist.

8. Shampoozusammensetzung nach einem vorangehenden Anspruch, worin die Gesamtmenge an Silikon 0,3 bis 5%, vorzugsweise 0,5 bis 3%, auf das Gewicht der Gesamtshampoozusammensetzung, ist.

9. Verwendung einer zusammensetzung, wie in einem der Ansprüche 1 bis 8 definiert, zum selektiven Konditionieren von Kombinationshaar.

## Revendications

1. Composition aqueuse de traitement capillaire qui est une composition de shampoing, comprenant, outre l'eau :
i) une silicone à fonctionnalité amine ; et
ii) des particules émulsionnées d'une silicone non soluble à fonctionnalité hydroxyle, et de 0,001 à 5 % en poids, sur la base du poids de la composition totale de shampoing, d'un polymère de dépôt cationique sélectionné parmi les dérivés cationiques de cellulose et les dérivés cationiques de guar.

2. Composition aqueuse de shampoing comprenant, outre l'eau :
i) au moins un tensioactif sélectionné parmi les tensioactifs anioniques, non ioniques, zwittérioniques ou amphotères, ou des mélanges de ceux-ci ;
ii) une silicone à fonctionnalité amine ; et
iii) des particules émulsionnées d'une silicone non soluble à fonctionnalité hydroxyle, et de 0,001 à 5 % en poids, sur la base du poids total de la composition de shampoing, d'un polymère de dépôt cationique sélectionné parmi les dérivés cationiques de cellulose et les dérivés cationiques de guar, dans lesquelles le rapport en poids entre la silicone à fonctionnalité amine (ii) et la silicone à fonctionnalité hydroxyle (iii) est de 1 : 2 ou inférieur.

3. Composition de shampoing selon la revendication 1, dans laquelle le rapport en poids entre la silicone à fonctionnalité amine et la silicone à fonctionnalité hydroxyle s'établit dans la plage allant de 1 : 3 à 1 : 8, de préférence aux alentours de 1 : 4.

4. Composition de shampoing selon l'une quelconque des revendications précédentes, dans laquelle la silicone à fonctionnalité amine a un % molaire de fonctionnalité amine se situant dans la plage allant d'environ 0,1 à environ 8,0 % en mole, de préférence d'environ 0,1 à environ 5,0 % en mole, mieux encore d'environ 0,1 à environ 2,0 % en mole.

5. Composition de shampoing selon l'une quelconque des revendications précédentes, dans laquelle la silicone à fonctionnalité amine est sous la forme d'une émulsion d'huile de silicone à fonctionnalité amine avec du tensioactif non ionique et/ou cationique, et dans laquelle la taille moyenne des particules de silicone à fonctionnalité amine dans la composition de shampoing est inférieure à 2 microns.

6. Composition de shampoing selon l'une quelconque des revendications précédentes, dans laquelle la silicone émulsionnée à fonctionnalité hydroxyle a une taille moyenne de particules dans la composition de shampoing qui est inférieure à 2 microns.

7. Composition de shampoing selon l'une quelconque des revendications précédentes, dans laquelle la silicone émulsionnée à fonctionnalité hydroxyle a une viscosité (de la silicone elle-même) qui est d'au moins 500.000 cst.

8. Composition de shampoing selon l'une quelconque des revendications précédentes dans laquelle la quantité totale de silicone va de 0,3 à 5 %, de préférence de 0,5 à 3 % en poids de la composition totale de shampoing.

9. Utilisation d'une composition selon l'une quelconque des revendications 1 à 8 pour le conditionnement sélectif des cheveux mixtes.
